# EUROPEAN PATENT APPLICATION

(11) **EP 4 417 161 A1**
(43) Date of publication of application: **21.08.2024**
(21) Application number: 22881236.8
(22) Date of filing: 21.09.2022
(51) Int. Cl.: A61C 9/00, A61B 5/00, G06T 1/00, G06T 17/00, G16H 30/00, G16H 50/50, A61B 18/20

(54) **DATA PROCESSING APPARATUS AND DATA PROCESSING METHOD**

(30) Priority: 12.10.2021 KR 20210134684; 24.08.2022 KR 20220106345
(71) Applicant: Medit Corp., Seoul 07207 (KR)
(72) Inventor: KO, Kinam, Seoul 07207 (KR); BAEK, Jongwoong, Seoul 07207 (KR); NOH, Jongcheol, Seoul 07207 (KR)
(74) Representative: Taor, Simon Edward William
(86) International application number: PCT/KR2022/014108
(87) International publication number: WO 2023/063607

(57) **Abstract**

Disclosed is a data processing method including identifying a type of an object, determining, based on the type of the object, whether to activate or deactivate a scan filtering mode, and obtaining 3D scan data on the object by activating or deactivating the scan filtering mode according to the determining.

## Description

### Technical Field

Embodiments disclosed herein relate to a data processing apparatus and a data processing method, and more particularly, to an apparatus and method for processing or manipulating oral cavity images.

### Background Art

3D scanners are used to obtain information about the oral cavities of patients. 3D scanners may be of a handheld type configured to be inserted into the patient's oral cavity and taken out of the patient's oral cavity or a table type configured to scan a plaster model placed on a table by rotating the table.

A data processing apparatus such as a PC connected to a 3D scanner may generate a 3D oral cavity image using raw data acquired by the 3D scanner.

In some cases, raw data acquired by a 3D scanner may include data about a tissue that is not needed by a user such as a dentist. When raw data contains unnecessary tissue data, it may be difficult to accurately align pieces of the raw data, and thus, an accurate 3D oral cavity image may not be obtained.

When raw data contains unnecessary data, a user may remove the unnecessary data through an additional trimming operation using a data processing apparatus. Alternatively, the user may rescan an object to obtain raw data not containing unnecessary tissue data. Alternatively, a data processing apparatus may have a filtering function to remove unnecessary tissue data in real time from raw data that is obtained by scanning an object using a 3D scanner, thereby preventing carrying out such cumbersome operations described above. However, depending on the types of objects, worse results may be obtained when filtering is used than when filtering is not used. For example, when an object from which raw data is obtained is a plaster model, a data processing apparatus may remove a region of the raw data by mistaking the region as an unnecessary tissue region, and in this case, a 3D oral cavity image of the plaster model may be incorrectly generated differently from the user's intention.

Thus, the user may manipulate the data processing apparatus to determine, depending on the type of an object to be scanned, whether to perform a filtering operation. The user may need to scan a plaster model while scanning the oral cavity of a patient or scan the oral cavity of a patient while scanning a plaster model. In this case, the user may change an object such as the oral cavity of a patient or a plaster model to a new object while scanning the object using a 3D scanner. Then, the user has to manipulate the data processing apparatus to determine again, depending on the type of the new object, whether to perform a filtering operation. This is cumbersome work. In addition, when the 3D scanner is a handheld-type intraoral scanner configured to be inserted into the oral cavity, the user may alternately touch the oral cavity of a patient and the data processing apparatus, which is not good for hygiene.

### Disclosure

### Technical Solution

According to an embodiment, a data processing method performed by a data processing apparatus may include identifying a type of an object, determining, based on the type of the object, whether to activate or deactivate a scan filtering mode, and obtaining 3D scan data on the object by activating or deactivating the scan filtering mode according to the determining.

According to an embodiment, the determining of, based on the type of the object, whether to activate or deactivate the scan filtering mode may include determining, based on the type of the object being a model, to deactivate the scan filtering mode.

### Description of Drawings

FIG. 1 is a diagram illustrating an oral cavity image processing system according to an embodiment.
FIG. 2 is a block diagram illustrating an internal structure of a data processing apparatus according to an embodiment.
FIG. 3 is a diagram illustrating an operation of identifying an object in an input image by using an artificial intelligence technique, according to an embodiment.
FIG. 4 is a diagram illustrating identifying an object in an input image by using an artificial intelligence technique according to an embodiment.
FIG. 5 is a diagram illustrating a screen output from a data processing apparatus according to an embodiment.
FIGS. 6A to 6D are diagrams illustrating 3D scan data acquired by a data processing apparatus based on various scan filtering modes according to embodiments.
FIG. 7 is a diagram illustrating a user interface screen displayed by a data processing apparatus to change a current activated or deactivated state of a scan filtering mode, according to an embodiment.
FIG. 8 is a flowchart illustrating a data processing method according to an embodiment.
FIG. 9 is a flowchart illustrating a data processing method according to an embodiment.
FIG. 10 is a flowchart illustrating a data processing method according to an embodiment.

### Mode for Invention

In an embodiment, determining of, based on the type of the object, whether to activate or deactivate a scan filtering mode may include determining, based on the type of an object not being a model, to activate the scan filtering mode.

In an embodiment, obtaining of 3D scan data on the object may include changing a current activated or deactivated state of the scan filtering mode based on the current activated or deactivated state of the scan filtering mode not corresponding to the determining, and obtaining 3D scan data on the object based on the changed activated or deactivated state of the scan filtering mode.

In an embodiment, a data processing method may further include outputting a user interface screen for changing the current activated or deactivated state of the scan filtering mode based on the current activated or deactivated state of the scan filtering mode not corresponding to the determining, wherein the user interface screen may include at least one of the type of the object, a message guiding changing of the activated or deactivated state of the scan filtering mode based on the type of the object, and a message guiding changing of a scan operation.

In an embodiment, the changing of the current activated or deactivated state of the scan filtering mode may include receiving a signal for changing the current activated or deactivated state of the scan filtering mode, the signal being input in response to the outputting of the user interface screen, and changing the current activated or deactivated state of the scan filtering mode based on the signal.

In an embodiment, identifying of the type of the object may include identifying the object as a specific class when a percentage of pixels identified as the specific class among all pixels included in a frame received from a scanner is greater than or equal to a reference value.

In an embodiment, the identifying of the type of the object may include identifying the object as the specific class when a percentage of frames identified as the specific class among a reference number of frames obtained after a scan operation starts is greater than or equal to a reference value.

In an embodiment, the scan filtering mode may include a plurality of activation modes depending on objects to be filtered, the obtaining of the 3D data on the object by activating the scan filtering mode may include obtaining filtered 3D scan data on the object in one activation mode among the plurality of activation modes, and the one activation mode among the plurality of activation modes may include at least one of an activation mode selected by a user, an activation mode selected by default, and an activation mode used in a previous scan operation.

In an embodiment, the obtaining of the filtered 3D scan data on the object in the one activation mode among the plurality of activation modes may include identifying pixels of a class to be filtered in the one activation mode among pixels included in a frame received from a scanner, and obtaining filtered 3D scan data on the object by removing pixels of the identified class.

In an embodiment, a data processing apparatus may include at least one processor configured to execute at least one instruction, wherein the at least one processor may execute the at least one instruction to perform identifying a type of an object, determining, based on the type of the object, whether to activate or deactivate a scan filtering mode, and obtaining 3D scan data on the object by activating or deactivating the scan filtering mode according to the determining.

In an embodiment, a computer-readable recording medium may have recorded thereon a program for executing a data processing method, and the data processing method may include identifying a type of an object, determining, based on the type of the object, whether to activate or deactivate a scan filtering mode, and obtaining 3D scan data on the object by activating or deactivating the scan filtering mode according to the determining.

The present specification clarifies the scope of the present application, and explains the principle of the present application and discloses embodiments, such that those of ordinary skill in the art to which the present application pertains may work the present application. The embodiments disclosed herein may be implemented in various forms.

In the drawings, like reference numerals refer to like elements. In the present specification, not all the elements of embodiments are described. That is, general elements in the technical field to which the present application pertains are not described, or the same description as that given in other embodiments is omitted. In the present specification, terms such as "part" or "portion" may be used to denote a part or portion that is implemented with hardware or software, and in embodiments, a plurality of parts or portions may be included in one unit or element, or a part or portion may include a plurality of units or elements. Hereinafter, operational principles and embodiments of the present application are described with reference to the accompanying drawings.

In the present specification, an image may refer to an image (hereinafter referred to as an "oral cavity image") of at least one tooth, the oral cavity including at least one tooth, or a plaster model of the oral cavity.

Furthermore, in the present specification, an image may refer to a 2D image of an object or a 3D oral cavity image expressing an object in three dimensions. A 3D oral cavity image may be generated by modeling the structure of the oral cavity in three dimensions based on raw data, and may thus be referred to as a 3D oral cavity model. In addition, a 3D oral cavity image may be referred to as a 3D scan model or 3D scan data.

Hereinafter, in the present specification, the term "oral cavity image" is used as a generic term for a model or image representing the oral cavity in two or three dimensions.

In the present specification, raw data or the like may be obtained using at least one camera to express an object in two or three dimensions. Specifically, raw data may refer to data obtained to generate an oral cavity image, and when an object is scanned using a 3D scanner, data obtained using at least one image sensor included in the 3D scanner may be raw data. Raw data may be 2D image data or 3D image data.

Hereinafter, embodiments are described with reference to the accompanying drawings.

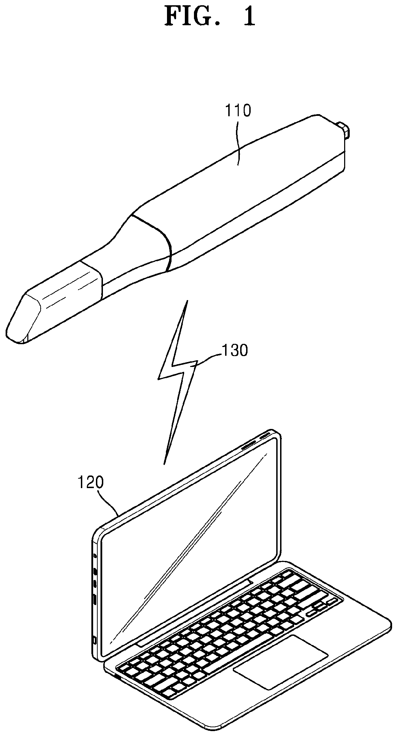

FIG. 1 is a view illustrating an oral cavity image processing system according to an embodiment.

Referring to FIG. 1, the oral cavity image processing system may include a 3D scanner 110 and a data processing apparatus 120 connected to the 3D scanner 110 through a communication network 130.

The 3D scanner 110 may be a device for acquiring images of an object.

In the present disclosure, an "object" may refer to an object to be scanned. The object may be a part of a body or may include a model modeled after a part of the body. The object may include an oral cavity, a plaster model or impression model of the oral cavity, an artificial structure insertable into the oral cavity, or a plaster or impression model or a dentiform of the artificial structure. For example, the object may include at least one of teeth and gingivae, a plaster model or impression model of at least one of teeth and gingivae, an artificial structure insertable into the oral cavity, and/or a plaster model or impression model of the artificial structure. Here, the artificial structure insertable into the oral cavity may include, for example, at least one of a correction device, an implant, a crown, an inlay, an onlay, an artificial tooth, and an orthodontic aid inserted into the oral cavity. In addition, the correction device may include at least one of a bracket, an attachment, an orthodontic screw, a lingual correction device, and a removable orthodontic maintenance device.

The 3D scanner 110 may acquire an image of at least one of the oral cavity, an artificial structure, and a plaster model, an impression model, or a dentiform of the oral cavity or the artificial structure. The 3D scanner 110 may be a handheld-type intraoral scanner which a user may hold and move with his/her hand to scan the oral cavity.

The 3D scanner 110 may be inserted into the oral cavity to scan teeth in a noncontact manner for acquiring an image of the oral cavity including at least one tooth. In FIG. 1, the 3D scanner 110 is illustrated as a handheld-type intraoral scanner, but is not limited thereto.

For example, the 3D scanner 110 may be a table scanner.

The 3D scanner 110 may acquire, as raw data, data on the surface of at least one object separated from teeth or gingivae in the oral cavity, artificial structures insertable into the oral cavity (for example, correction devices such as brackets and wires, implants, artificial teeth, orthodontic aids insertable into the oral cavity, etc.), and models such as plaster models, impression models, and dentiforms in order to image the surface of the at least one object.

The 3D scanner 110 may transmit the raw data to the data processing apparatus 120 through the communication network 130.

The data processing apparatus 120 may be connected to the 3D scanner 110 through the communication network 130 which is a wireless or wired communication network. The data processing apparatus 120 may be any electronic apparatus capable of receiving raw data from the 3D scanner 110 and generating, processing, displaying, and/or transmitting an oral cavity image based on the received raw data. For example, the data processing apparatus 120 may be a computing apparatus such as a smartphone, a laptop computer, a desktop computer, a PDA, or a tablet PC, but is not limited thereto. In addition, the data processing apparatus 120 may a server (or server device) configured to process oral cavity images.

The data processing apparatus 120 may generate a 3D oral cavity image, that is, 3D scan data, based on the raw data received from the 3D scanner 110. The data processing apparatus 120 may display the 3D oral cavity image on a display, or may output or transmit the 3D oral cavity image to an external device.

In another example, the 3D scanner 110 may acquire raw data by scanning the oral cavity, process the raw data to generate 3D information, and transmit the 3D information to the data processing apparatus 120.

In an embodiment, the 3D scanner 110 may acquire 3D information about an object in various manners. For example, the 3D scanner 110 may acquire 3D information about an object by a confocal method. In another example, the 3D scanner 110 may acquire 3D information about an object by an optical triangulation method. However, these are merely examples. The 3D scanner 110 may acquire 3D information from raw data by using various methods other than the confocal method or the optical triangulation method and may transmit the 3D information to the data processing apparatus 120.

The data processing apparatus 120 may analyze, process, manipulate, and/or display the received 3D information, and/or transmit the received 3D information to an external device.

In an embodiment, the data processing apparatus 120 may generate 3D scan data on an object by using images, that is, frames, of the object that are received from the 3D scanner 110.

In an embodiment, when the data processing apparatus 120 generates 3D scan data, the data processing apparatus 120 may activate a scan filtering mode to obtain 3D scan data from which unnecessary data is removed.

According to an embodiment, in the scan filtering mode, unnecessary data may be removed from frames received from the 3D scanner 110. In an embodiment, unnecessary data may refer to tissue data that a user does not want to include in 3D scan data.

In an embodiment, when the scan filtering mode is active, the data processing apparatus 120 may filter frames received from the 3D scanner 110 to remove regions corresponding to unnecessary data.

In an embodiment, the data processing apparatus 120 may determine whether to activate the scan filtering mode.

In some cases, worse results may be obtained when the data processing apparatus 120 performs scan filtering on an image of an object than when the data processing apparatus 120 does not perform scan filtering on the image of the object. For example, when an object is a model such as a dentiform, a plaster model, or an impression model, the data processing apparatus 120 may remove a region of an image of the object by mistaking the region as an unnecessary tissue region, and in this case, 3D scan data on the model may be incorrectly generated differently from user's intention.

In an embodiment, the data processing apparatus 120 may determine, depending on the type of an object, whether to activate the scan filtering mode. To this end, the data processing apparatus 120 may recognize the object and identify the type of the object.

In an embodiment, the data processing apparatus 120 may determine, depending on the type of an object, whether to activate the scan filtering mode and may obtain 3D scan data on the object by activating or deactivating the scan filtering mode according to the determination.

In an embodiment, the data processing apparatus 120 may obtain/acquire 3D scan data on the object by automatically activating or deactivating the scan filtering mode according to the determination. Alternatively, in some embodiments, when it is needed to change the activation or deactivation state of the scan filtering mode according to the determination, the data processing apparatus 120 may output a user interface screen to guide changing the activation or deactivation state of the scan filtering mode, change the activation or deactivation state of the scan filtering mode based on a signal input by a user in response to the user interface screen, and obtain 3D scan data on the object.

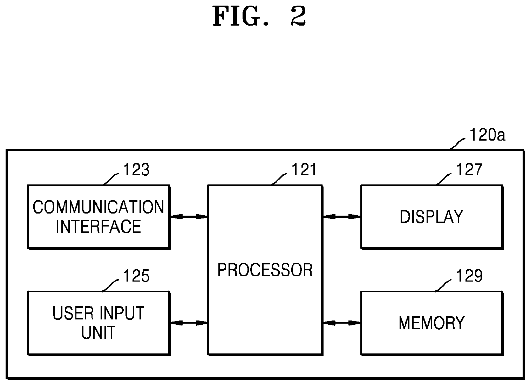

FIG. 2 is a block diagram illustrating the internal structure of a data processing apparatus 120a according to an embodiment.

In an embodiment, the data processing apparatus 120a may also be referred to as an oral cavity image processing apparatus.

The data processing apparatus 120a shown in FIG. 2 may be an embodiment of the data processing apparatus 120 shown in FIG. 1. Therefore, the same description as the description of the data processing apparatus 120 shown in FIG. 1 is omitted here.

Referring to FIG. 2, the data processing apparatus 120a may include a processor 121, a communication interface 123, a user input unit 125, a display 127, and a memory 129.

The data processing apparatus 120a may generate, process, manipulate, display, and/or transmit a 3D oral cavity model by using images and/or 3D information on an object that are received from the 3D scanner 110. Alternatively, the data processing apparatus 120a may receive a 3D oral cavity model from an external server or an external device through a wired or wireless communication network.

In an embodiment, the memory 129 may store at least one instruction. The memory 129 may store at least one instruction or program executable by the processor 121.

In an embodiment, the memory 129 may store data received from the 3D scanner 110, such as images or 3D information obtained by scanning the oral cavity or an oral cavity model. The memory 129 may store point location information and point connection relationship information of 3D oral data received from the 3D scanner 110.

In an embodiment, the memory 129 may include at least one instruction for generating a 3D oral cavity model of an object based on an image of the object.

In an embodiment, the memory 129 may store dedicated software executable in connection with the 3D scanner 110. Here, the dedicated software may refer to a dedicated program or application. When the data processing apparatus 120a operates in connection with the 3D scanner 110, the dedicated software stored in the memory 129 may be connected to the 3D scanner 110 and may receive, in real time, data such as images acquired by scanning an object. The dedicated software may provide a menu bar including various menus or tools on the display 127 such that a user may use data acquired by the 3D scanner 110. The menu bar provided by the dedicated software may be output together with 3D scan data on an object.

In an embodiment, the dedicated software may include instructions, a dedicated program, a dedicated tool, a dedicated application, or the like for filtering a predetermined region when generating a 3D oral cavity model from an image of an object. The dedicated software may filter a predetermined region in an image acquired from the 3D scanner 110 based on a scan filtering activation mode and generate 3D scan data from which unnecessary data is removed.

In an embodiment, class information may be stored in the memory 129. In an embodiment, the class information may refer to information for identifying the class of an object included in an image. The object may include the oral cavity, an artificial structure insertable into the oral cavity, or a model such as a plaster model, an impression model, or a dentiform of the oral cavity or the artificial structure. In an embodiment, class information on an object may be information indicating, depending on the type of the object, at least one of the tongue, gingivae, soft tissue, blood, saliva, teeth, palates, lips, abutments, metals, correction devices, gypsum, impressions, gloves, human hands, and cheeks. However, this just an embodiment, and objects and class information on the objects are not limited thereto.

In an embodiment, the class information stored in memory 129 may include numbers respectively corresponding to classes. For example, when an object is a tooth, class information corresponding to the tooth may include a unique number 0 representing teeth. Similarly, when an object is a gingiva, class information corresponding to the gingiva may include a unique number 1 representing gingivae.

In an embodiment, the memory 129 may store information about whether to activate the scan filtering mode according to the type of an object. For example, the memory 129 may store information indicating that the scan filtering mode is to be deactivated when the type of an object is a model. Additionally, the memory 129 may store information indicating that the scan filtering mode is to be activated when the type of an object is not a model.

In an embodiment, the scan filtering mode may include a plurality of activation modes. In an embodiment, the activation modes may be classified according to targets to be filtered. For example, the activation modes may include a first activation mode, a second activation mode, and a third activation mode, and in an embodiment, the memory 129 may store class information corresponding to regions be filtered according to the activation modes. For example, when the scan filtering mode is the third activation mode, the memory 129 may store information indicating that all classes corresponding to tissues other than teeth are to be filtered.

In an embodiment, the data processing apparatus 120a may include the processor 121 to execute the at least one instruction. The processor 121 may include one or a plurality of processors. For example, the processor 121 may execute the at least one instruction to control at least one element of the data processing apparatus 120a and thus to perform an intended operation. Therefore, even when the processor 121 is described as performing a certain operation, this may mean that the processor 121 controls at least one element of the data processing apparatus 120a to perform the certain operation.

Here, the at least one instruction may be stored in the memory 129 of the data processing apparatus 120a which is separate from the processor 121, or may be stored in an internal memory (not shown) of the processor 121.

In an embodiment, the at least one instruction may include an instruction for determining, based on the type of an object, whether to activate the scan filtering mode.

In an embodiment, the at least one instruction may be an instruction for determining, based on the type of an object, whether to activate the scan filtering mode and acquiring 3D scan data on the object by activating or deactivating the scan filtering mode according to the determination.

In an embodiment, the processor 121 may recognize an object. In an embodiment, the processor 121 may identify the type of the object. In an embodiment, the processor 121 may identify the type of the object by analyzing a frame received from the 3D scanner 110. For example, the processor 121 may identify the classes of pixels included in the frame and identity the class of the frame or the object included in the frame based on whether the percentage of pixels identified as a specific class among all the pixels included in the frame is greater than or equal to a reference value.

In an embodiment, the processor 121 may identify the class of the object based on a reference number of frames. In an embodiment, the processor 121 may identify the type of the object using a reference number of frames among frames received from the 3D scanner 110. For example, the reference number of frames may be 60 frames that are first received from the 3D scanner 110 in a scan operation, but is not limited thereto. In an embodiment, the processor 121 may identify the class of the object based on whether the number of frames identified as a specific class among the reference number of frames is greater than or equal to a reference value.

In an embodiment, the processor 121 may determine, based on the type of an object, whether to activate or deactivate the scan filtering mode. In an embodiment, scan filtering may refer to an operation of removing unnecessary data that a user does not want from an image received from the 3D scanner 110. In an embodiment, the scan filtering mode may refer to a mode in which a scan filtering operation is performed to remove unnecessary data from an image of an object.

In an embodiment, the processor 121 may determine, based on the type of an object, whether to activate or deactivate the scan filtering mode.

In an embodiment, when the processor 121 determines to activate the scan filtering mode, filtered 3D scan data may be obtained by removing unnecessary data from the image in the scan filtering mode.

In an embodiment, when the processor 121 determines to deactivate the scan filtering mode, the processor 121 may deactivate the scan filtering mode and obtain 3D scan data without filtering the image.

In an embodiment, when the object is an object in the oral cavity of a patient, for example, teeth or an artificial structure inserted in the oral cavity, the processor 121 may determine to activate the scan filtering mode.

The oral cavity of a patient may include soft and hard tissues. Hard tissue may refer to hard tissue such as alveolar bone or teeth. Soft tissue may refer to soft tissue such as gingivae, mucosa, lips, and tongues, excluding bone or cartilage. Gingivae refers to parts of mucous membranes that cover teeth and roots of teeth and may also be referred to as gums. In addition, the oral cavity of a patient may contain blood or saliva.

When the teeth of a patient are the subject of dental diagnosis and treatment, a user may want to use 3D scan data on only the teeth. However, tissues included in the oral cavity other than the teeth may affect the 3D scan data on the teeth. For example, when the patient moves his/her tongue or lips while the oral cavity of the patient is scanned, the 3D scan data on the teeth may include data about the tongue or lips. In addition, gingivae or soft tissue may degenerate, affecting scan data on teeth. For example, when the gingivae or other soft tissue swells due to the oral cavity state of a patient or the administration of medication, the soft tissue may block the teeth or move relative to the teeth, and thus, accurate scan data on the teeth may not be obtained. In addition, when blood or saliva inside the oral cavity accumulates around the teeth, accurate scan data about the teeth may not be obtained.

Therefore, to obtain accurate scan data on the teeth, it is necessary to minimize data in unnecessary regions that may affect the teeth.

In an embodiment, when an object is the oral cavity of a patient, the processor 121 may activate the scan filtering mode to prevent unnecessary data from affecting the generation of 3D scan data on teeth.

In an embodiment, the scan filtering mode may include a plurality of activation modes depending on targets or regions to be filtered. For example, the activation modes may be classified into three modes depending on regions to be filtered.

In an embodiment, a first activation mode may refer to a mode for filtering out soft tissue other than teeth, gingivae, and palate. The first activation mode may be a mode that filters tissue excluding teeth, gingivae, and palate.

In an embodiment, a second activation mode may refer to a mode for obtaining scan data only on teeth and gingivae. The second activation mode may be a mode for filtering out soft tissue and leaving only teeth and gingivae.

In an embodiment, a third activation mode may be a mode for removing all tissues other than teeth.

In an embodiment, the processor 121 may acquire 3D scan data on an object by activating the scan filtering mode. The processor 121 may use one of the activation modes to acquire filtered 3D scan data on the object.

In an embodiment, the processor 121 may receive a user's selection of one activation mode among a plurality of activation modes. Alternatively, in an embodiment, the processor 121 may select one activation mode among a plurality of activation modes as a default. Alternatively, in an embodiment, the processor 121 may remember one of the activation modes that is used in the previous scan operation and automatically select the activation mode that is used in the previous scan operation.

In an embodiment, the processor 121 may identify pixels of a class to be filtered among pixels included in a frame received from a scanner according to a selected activation mode.

In an embodiment, the processor 121 may obtain filtered 3D scan data on the object by removing the identified pixels.

In an embodiment, the processor 121 may deactivate the scan filtering mode when the type of an object to be scanned is a model. When the type of an object is a model, the object may be, for example, a plaster model, an impression model, a dentiform, or the like.

When the scan filtering mode is deactivated, a data filtering operation may not be performed during the generation of scan data from an image of the object.

Unlike the oral cavities of patients, plaster models, impression models, or dentiforms that imitate the oral cavity or artificial structures do not contain unnecessary tissue that may affect the generation of scan data on an object. In other words, unlike the oral cavities of patients, plaster models or impression models do not include tongues, palates, or lips, and are free from phenomena such as swelling of gingivae or accumulation of blood or saliva on teeth. That is, plaster models or impression models do not include data that affects the generation of scan data on an object. When a plaster model or an impression model is scanned in a state in which the scan filtering mode is activated, there is a possibility that a portion of the entire area to be acquired from the plaster model or the impression model may be mistaken as a portion to be filtered and removed, and thus, inaccurate 3D scan data may be obtained.

More accurate 3D scan data may be obtained from an object by using more images of the object. Thus, in an embodiment, when the type of an object is a model such as a plaster model or an impression model, or a dentiform, the processor 121 may deactivate the scan filtering mode and obtain scan data on the object without filtering.

In an embodiment, the processor 121 may identify the type of an object by identifying a scan step. For example, when the scan step is an impression scan operation, the processor 121 may identify the type of the object as an impression model. In an embodiment, the impression scan operation may refer to an operation of scanning an impression model to generate 3D scan data on the impression model. The impression model is a model shaped after the appearance of hard and soft tissues of the oral cavity including teeth and is a negative imprint of the actual oral cavity of a patient, and thus, the impression scan operation includes converting the scanned negative imprint into a relief model.

In an embodiment, when the scan step is an impression scan operation, the processor 121 may identify the type of the object as an impression model and thus deactivate the scan filtering mode to obtain 3D scan data on the impression model without filtering.

In an embodiment, the processor 121 may identify the current activated or deactivated state of the scan filtering mode. In an embodiment, when the current activated or deactivated state of the scan filtering mode does not correspond to an activated or deactivated state of the scan filtering mode that is determined based on the type of an object, the processor 121 may automatically change the current activated or deactivated state of the scan filtering mode according to the type of the object.

For example, when the type of the object identified by the processor 121 is a plaster model, the processor 121 may determine that the scan filtering mode is to be deactivated. When the scan filtering mode is currently in the activated state, the processor 121 may automatically change the state of the scan filtering mode from the activated state to the deactivated state. The processor 112 may acquire 3D scan data on the plaster model in a state in which the scan filtering mode is deactivated.

In an embodiment, when the current activated or deactivated state of the scan filtering mode does not correspond to an activated or deactivated state of the scan filtering mode that is determined based on the type of an object, the processor 121 may not automatically change the current activated or deactivated state of the scan filtering mode but may inform a user that the current activated or deactivated state of the scan filtering mode does not correspond to the type of the object. For example, the processor 121 may output a user interface screen for changing the activated or deactivated state of the scan filtering mode. The processor 121 may guide the user to change the activated or deactivated state of the scan filtering mode by outputting a user interface screen for changing the activated or deactivated state of the scan filtering mode.

In an embodiment, the processor 121 may receive a signal for changing the activated or deactivated state of the scan filtering mode from the user in response to the output of the user interface screen. In an embodiment, the processor 121 may change the current activated or deactivated state of the scan filtering mode according to the type of the object based on the signal input by the user for changing the activated or deactivated state of the scan filtering mode.

In an embodiment, the display 127 may output a 3D oral cavity model and various menu bars for using or editing the 3D oral cavity model. In an embodiment, the menu bars may include a menu for selecting whether to activate the scan filtering mode. In addition, the menu bars may include a menu for selecting one of the first activation mode, second activation mode, and third activation mode.

In an embodiment, the display 127 may output a user interface screen for changing the current activated or deactivated state of the scan filtering mode. The user interface screen may include at least one of an identified type of an object, a message for a user to change the scan filtering mode to the activated or deactivated state according to the object, and a message for a user to change a scan step/operation.

In an embodiment, the communication interface 123 may communicate with at least one external electronic device through a wired or wireless communication network. For example, the communication interface 123 may communicate with the 3D scanner 110 under control by the processor 121. In an embodiment, the communication interface 123 may receive images from the 3D scanner 110 or obtain 3D information. In an embodiment, the communication interface 123 may acquire a scan model by communicating with a device such as an external electronic device or an external server other than the 3D scanner 110.

The communication interface 123 may include at least one short-distance communication module capable of performing communication according to communication references such as Bluetooth, Wi-Fi, Bluetooth Low Energy (BLE), NFC/RFID, Wifi Direct, UWB, or ZIGBEE.

In addition, the communication interface 123 may further include a remote communication module capable of communicating, according to a long-distance communication reference, with a server that supports long-distance communication. For example, the communication interface 123 may include a remote communication module capable of performing communication through a network for Internet communication. For example, the communication interface 123 may include a remote communication module capable of performing communication through a communication network conforming to a communication reference such as 3G, 4G, and/or 5G.

In addition, the communication interface 123 may perform wired communication with the 3D scanner 110, an external server, an external electronic device, and the like. To this end, the communication interface 123 may include at least one port for connection with the 3D scanner 110 or an external electronic device through a cable. The communication interface 123 may communicate with the 3D scanner 110 or an external electronic device that is connected to the at least one port of the communication interface 123 through a cable.

In an embodiment, the user input unit 125 may receive a user's input for controlling the data processing apparatus 120a. The user input unit 125 may include a user input device such as a touch panel for sensing a user's touch, a button for receiving a user's push manipulation, or a mouse or keyboard for designating or selecting a point on a user interface screen. However, the user input unit 125 is not limited thereto. In addition, the user input unit 125 may include a voice recognition device for voice recognition. For example, the voice recognition device may be a microphone and may receive a user's voice command or voice request. Then, the processor 121 may control the data processing apparatus 120a to perform an operation corresponding to the voice command or voice request.

In an embodiment, the user input unit 125 may receive a command ordering the activation or deactivation of the scan filtering mode from a user such as a dentist.

In an embodiment, the user input unit 125 may receive, from a user, a command ordering a change of the current activated or deactivated state of the scan filtering mode.

In an embodiment, the user input unit 125 may receive, from a user, a command ordering an operation in one activation mode among a plurality of scan filtering modes.

In an embodiment, the user input unit 125 may receive from a user, a command ordering a change a scan operation to an impression scan operation.

As described above, according to embodiments, the data processing apparatus 120a may determine, based on the type of an object, whether to activate or deactivate the scan filtering mode and generate scan data on the object according to the determination. Thus, more accurate 3D scan data on the object may be obtained according to the type of the object.

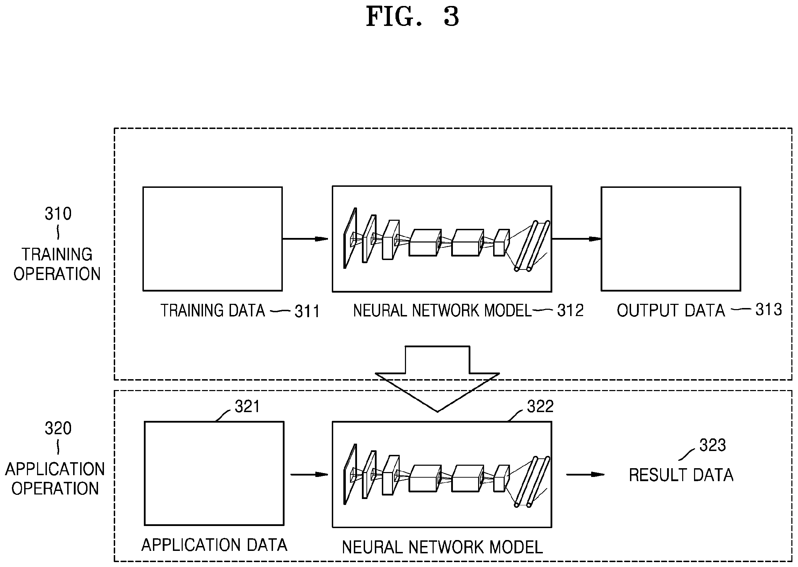

FIG. 3 is a diagram illustrating an operation of identifying an object in an input image by using artificial intelligence (Al) techniques according to an embodiment.

In an embodiment, the data processing apparatus 120 may identify an object in a frame received from the 3D scanner 110 by using Al techniques. The Al techniques may include machine learning (deep learning) techniques and element techniques using the machine learning techniques. The Al techniques may be implemented using algorithms. Here, an algorithm or a set of algorithms for implementing the Al technique is referred to as a neural network or a neural network model. The neural network may receive input data, perform calculations for analysis and classification, and output result data.

Referring to FIG. 3, the operation of identifying an object in an input frame by using Al techniques may generally include two operations. The two operations may include a training operation 310 for training a learning model and an application operation 320 for applying the trained learning model.

First, in the training operation 310, a neural network, that is, a neural network model 312, may be trained using a plurality of pieces of training data 311 as an input. The training data 311 for training the neural network model 312 may be obtained from a training DB.

The training DB may include training images of objects generated in various surrounding environments. The training images of objects obtained from the training DB and used as the training data 311 may be captured using various types of 3D intraoral scanners and transmitted to a data processing apparatus or the like through a communication network.

In an embodiment, the training images may include one or more objects. Examples of various objects may include the oral cavity, an artificial structure insertable into the oral cavity, or a model such as a plaster model, an impression model, or a dentiform of the oral cavity or the artificial structure. More specifically, objects may include at least one of tongues, gingivae, soft tissue, blood, saliva, teeth, palates, lips, abutments, metals, correction devices, gypsum, impressions, gloves, human hands, and cheeks. However, those are just examples, and objects are not limited to the types listed above. Types of objects may include only some of the types listed above or may include other types in addition to the types listed above.

A large amount of training data is required to obtain a more accurate learning model. When the amount of training data 311 is insufficient, the learning model trained with the insufficient training data 311 is likely to have poor performance.

In an embodiment, a manufacturer of the data processing apparatus 120 and/or the neural network model 312 may generate additional images based on training images and use the additional images as the training data 311. The manufacturer may analyze images generated by various types of 3D scanners and generate new additional images based on the images to increase the amount of training data to be used for designing the neural network model 312

In an embodiment, the manufacturer may interpolate training images to generate additional images. For example, when the training images include images obtained by a 3D scanner scanning an object when the distance between the 3D scanner and a point on the object is 1 cm and 2 cm, the manufacture may perform an interpolation on the two images to obtain an interpolation image that may be captured when the distance between the 3D scanner and the point of the object is 1.5 cm.

In an embodiment, the manufacturer may modify training images to generate additional images. For example, the manufacturer may modify training images to generate additional images of various types by using various methods such as a method of adjusting the colors, brightness, and contrast of training images, a method of adjusting the resolution of training images, or a method of blurring training images to adjust sharpness, or a method of adding noises to training images.

In an embodiment, the training data 311 may include training images and a segmentation mask for the training images. The segmentation mask may be a mask for labeling the types of objects, that is, the classes of objects, on the basis of pixels of training images. The segmentation mask may be a mask for dividing training images on the basis of pixels to indicate, in the training images, the positions and shapes of objects, the relationship between objects, which pixels belong to which objects, or the like, and assigning a label to each pixel of the training images.

In an embodiment, the training data 311 may include, as a training data set, training images for an object obtained from the training DB and a segmentation mask for the training images.

The training data 311 may be input to the neural network model 312.

In an embodiment, the neural network model 312 may be trained using the training data 311 including training images for objects generated in various environments and segmentation masks for the training images.

In an embodiment, the neural network model 312 may be a convolution neural network (CNN), a deep convolution neural network (DCNN), or a CapsNet neural network.

In an embodiment, the neural network model 312 may be trained such that the neural network model 312 may find or lean methods such as a method of receiving various pieces of data and analyzing the received data, a method of classifying the received data, and/or a method of extracting, from the received data, features necessary for generating result data.

In an embodiment, the neural network model 312 may be trained to analyze the training data 311 to identify the color, shape, type, location, size, or the like of each object included in training images.

The neural network model 312 may be created as an Al model having desired characteristics by applying a learning algorithm to a large amount of training data. This training may be performed by the data processing apparatus 120 or may be performed by a separate server/system. Here, the learning algorithm refers to a method of training a target device (for example, a robot) using a large amount of training data to enable the target device to make decisions or predictions by itself.

Examples of the learning algorithm include supervised learning, unsupervised learning, semi-supervised learning, and reinforcement learning. In embodiments, the learning algorithm is not limited to the examples listed above unless otherwise specified.

For example, the neural network model 312 may be trained as a data inference model by a supervised learning method using training data as an input. Alternatively, the neural network model 312 may learn on its own the type of data needed to identify an object included in an image without any guidance and may thus be trained as a data inference model by an unsupervised learning method of finding references for identifying objects included in images. Alternatively, the neural network model 312 may be trained as a data inference model by a reinforcement learning method in which whether results of inferring an object included in an image are correct or not is fed back.

In an embodiment, the neural network model 312 may include an input layer, a hidden layer, and an output layer. In an embodiment, the hidden layer may include a plurality of hidden layers. The neural network model 312 may be a deep neural network (DNN) including two or more hidden layers. The DNN may refer to a neural network configured to perform operations through a plurality of layers, and the depth of the DNN may increase according to the number of internal layers that perform operations. DNN operations may include CNN operations or the like.

In an embodiment, the neural network model 312 may be designed in many different manners depending on a method of implementing the neural network model 312, the accuracy and reliability of results by the neural network model 312, or the speed and capacity of a processor.

In an embodiment, the neural network model 312 may be a neural network based on a dilated convolution (or atrous convolution) method.

Contextual information is important to accurately identify an object included in an image. Contextual Information may refer to information that indicates, for example, the environment in which the background of an object is, the types of other objects around an object, or the positions of objects or a relationship between objects. Securing sufficient contextual information is necessary to identify an object, and to this end, it is necessary to consider a relatively wide receptive field. In general, examples of a method of expanding the receptive field in a CNN include expanding the size of a kernel and increasing the number of convolutional layers, but these methods greatly increase the amount of computation.

In an embodiment, the neural network model 312 may use an atrous convolution-based neural network. Atrous convolution refers to a convolution technique for increasing the view of a convolution by leaving gaps between pixels accepted by a convolution filter. When an atrous convolution-based neural network is used, the number of input pixels does not vary, but inputs are accepted in a wider range, making it possible to consider the characteristics of surrounding pixels.

Atrous convolution-based neural networks introduce a dilation rate as a parameter defining a gap in a kernel of a convolution layer. For example, a 3x3 kernel with a dilation rate of 2 uses nine parameters and thus has the same view as a 5x5 kernel.

In the present disclosure, a wide view is required to segment an input image that is input from the 3D scanner 110 in real time, and when there is no room to use a convolution including a plurality of layers or a large kernel, the data processing apparatus 120 may increase the receptive field with a small amount of calculation by using an atrous convolution. The atrous convolution increases the receptive field by adding zero padding to the inside of a filter, and a weight for a gap in a kernel is filled with 0. The term "receptive field" refers to an area that a filter looks at a time. The use of a wide receptive field is advantageous in identifying and extracting features of an image in an area that a filter looks at a time.

In an embodiment, the neural network model 312 may be an algorithm that extracts features by analyzing and classifying input data using a plurality of pieces of training data as input values. The neural network model 312 may be a model trained to identify an object from input data. To this end, the neural network model 312 may learn how to detect the color, shape, type, location, size, or the like of an object in a training image.

In an embodiment, the neural network model 312 may learn and/or may be trained how to identify an object included in an image of a plurality of pieces of training data 311 in response to the input of the plurality of pieces of training data 311 and may be creased based on results of the learning and/or training.

In an embodiment, the neural network model 312 may identify the type of an object included in an image by considering semantic information on the object. Semantic information may refer to the color or shape of an object, the location of an object, the size of an object, a positional relationship or size relationship between objects, or the like.

In an embodiment, the neural network model 312 may learn types of objects and a spatial relationship between objects.

In an embodiment, the neural network model 312 may be trained to learn a relationship between a training image and a segmentation mask and identify an object in the training image.

In an embodiment, the neural network model 312 may be trained using the segmentation mask for the training image as a correct answer set. The neural network model 312 may infer/predict the type or location of an object, the types or locations of surrounding objects, the size of an object, or the like from input training images and may be repeatedly trained such that results of prediction are the same as the correct answer set.

To increase the accuracy of results output through the neural network model 312, output data 313, that is, results of each training operation, is fed back to the neural network model 312 and is used to update weights of the neural network model 312.

In an embodiment, the neural network model 312 may obtain, as a loss function, the difference between a ground truth and the output data 313 output from the output layer. The ground truth may refer to a segmentation mask labeling objects included in training images according to the types of the objects. The neural network model 312 may receive the loss function again and continuously modify weight values for edges included in the hidden layer to minimize the loss function. The weight values for edges may be optimized through iterative training and may be repeatedly modified until the accuracy of results satisfies a predetermined level of reliability. The neural network model 312 may be formed by weight values that are finally set for edges.

The neural network model 312 with the weight values finally set for edges may be embedded in the data processing apparatus 120 and may be used to identify objects from input images.

An operation of learning how to obtain the types of objects from images by using the neural network model 312 may be performed in the data processing apparatus 120. Alternatively, the learning operation may be performed in an external computing device that is separate from the data processing apparatus 120. For example, the operation of learning how to obtain the types of objects from images by using the neural network model 312 may require a relatively complex amount of calculation. Therefore, the external computing device may perform the learning operation, and the data processing apparatus 120 may receive the learned neural network model 312 from the external computing device after the learning operation is finished, thereby reducing the amount of calculation of the data processing apparatus 120. The data processing apparatus 120 may receive the neural network model 312 from an external server and may store the neural network model 312 in the memory 129.

In the application operation 320, application data 321 may be input to a trained neural network model 322, and result data 323 indicating the types of objects included in the application data 321 may be obtained.

In an embodiment, the application data 321 may refer to an image input in real time from the 3D intraoral scanner 110, and the result data 323 may refer to information indicating the type or location of an object included in the image. For example, the result data 323 output from the neural network model 322 may refer to a segmentation mask indicating a label for each pixel of the object included in the application data 321. The segmentation mask output as the result data 323 may indicate the location and shape of the object, the relationship between a plurality of objects, the classes of pixels, or the like.

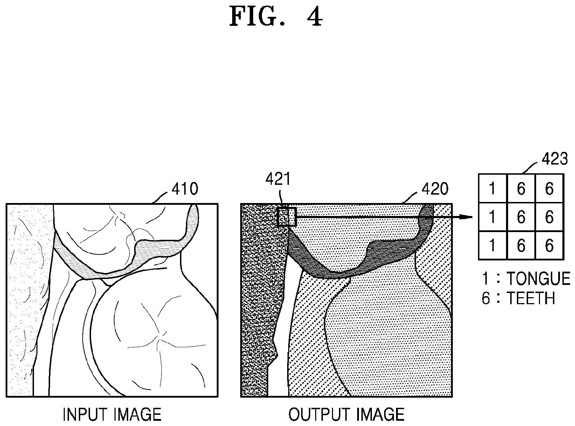

FIG. 4 is a diagram illustrating identifying an object in an input image 410 using an AI technique according to an embodiment.

Referring to FIG. 4, the neural network model 322 described with reference to FIG. 3 may receive an input image 410 and obtain an output image 420 indicating features of an object included in the input image 410.

A user may scan an object using the 3D scanner 110. The 3D scanner 110 may transmit, in real time, images obtained by photographing the object to the data processing apparatus 120 through the communication network 130.

In an embodiment, the data processing apparatus 120 may input the input image 410 received from the 3D scanner 110 to the neural network model 322.

In an embodiment, the neural network model 322 included in the data processing apparatus 120 may extract features from the input image 410 by analyzing and classifying the input image 410. For example, the neural network model 322 may analyze the input image 410 to obtain features such as the type, size, and positional relationship of one or more objects included in the input image 410.

In an embodiment, the neural network model 322 may identify an object included in the input image 410 based on features extracted from the input image 410. For example, the neural network model 322 may analyze the features extracted from the input image 410 to identify the type and location of an object included in the input image 410.

In an embodiment, the output image 420 may be in the form of a segmentation mask indicating the classes of objects by showing the class of each pixel of the input image 410.

In an embodiment, the segmentation mask may indicate the type of an object included in the input image 410 in units of pixels. The neural network model 322 may express the position and shape of an object included in the input image 410, which pixel belongs to which object, or the like by assigning labels respectively to the pixels of the input image 410.

In an embodiment, the segmentation mask may include pixel-based labels, that is, pixel-based class information. The pixel-based class information may be expressed by a color for each pixel or a unique number for each pixel. Each pixel color and each pixel number may correspond to each other. For example, a specific color corresponding to a specific class may correspond to a specific number.

In FIG. 4, when a predetermined area 421 included in the output image 420 is enlarged, the enlarged predetermined area 421 may be expressed like a table 423 shown on the right side of the output image 420. The table 423 may be a matrix indicating identified classes of pixels included in the predetermined area 421 by using numbers. For example, referring to FIG. 4, when the tongue and gingivae are included in the predetermined area 421, the table 423 corresponding to the predetermined area 421 may include numbers assigned to the pixels and indicating the classes of the tongue and gingivae. Referring to FIG. 4, when number 1 indicates the tongue and numbers indicates the gingivae among numbers indicating the classes of objects, identified class information on nine pixels of the predetermined area 421 is expressed with number 1 and number 6 in the table 423.

In an embodiment, the data processing apparatus 120 may use the output image 420 obtained from the neural network model 322 to identify the classes of objects included in the input image 410 and relative positions of the objects.

In an embodiment, the data processing apparatus 120 may obtain the percentage of pixels identified as being included in a predetermined class among all of the pixels, and when the percentage of pixels identified as being included in the predetermined class is greater than or equal to a reference value, the input image 410 may be identified as the predetermined class.

For example, the data processing apparatus 120 may obtain, using the segmentation mask, information that the percentage of pixels identified as the tongue, the percentage of pixels identified as teeth, and the percentage of pixels identified as gingivae among all of the pixels are 16%, 61%, and 17%, respectively. Among the classes, the data processing apparatus 120 may identify, as teeth, the class having a percentage of greater than a reference value of 60% and determine the entire class of the input image 410 as teeth.

In some cases, it may be difficult for the neural network model 322 to accurately determine the types of objects by using just one frame. In an embodiment, the neural network model 322 may determine the types of objects using a predetermined number of frames.

In an embodiment, when the neural network model 322 identifies the type of an object included in a frame, the neural network model 322 may consider the color, shape, and size of the object in the frame together with the shapes or colors of objects located around the object in the frame. For example, it is difficult to distinguish soft tissues such as gingivae and mucous membranes from each other based on only the colors thereof. In an embodiment, when the neural network model 322 determines whether an object is a mucous membrane or a gingiva, the neural network model 322 may consider how far the object is from teeth and/or the colors, shapes, or classes of surrounding pixels such as the shape of the object and the shapes of objects around the object. To this end, as described above, the neural network model 322 may use an atrous convolution-based neural network. For example, instead of extracting features of an object using a 3X3 filter that is mainly used by general CNN-based neural networks, the neural network model 322 may place spaces having a weight of 0 in a kernel around a target pixel to consider features of a pixel located in the center of the kernel and features of pixels surrounding the center pixel at a distance from the center pixel for statistically calculating the probability that the center pixel corresponds to a gingiva or a mucous membrane.

In an embodiment, the data processing apparatus 120 may identify the type of an object based on only a reference number of frames. For example, when the percent of frames identified as gingivae among a reference number of frames, for example, 60 frames, received from the 3D scanner 110 after the 3D scanner 110 start scanning is greater than or equal to a reference value, for example, 70 %, the data processing apparatus 120 may determine that an object that is currently scanned by the 3D scanner 110 is a gingiva.

In an embodiment, the data processing apparatus 120 may identify the type of an object using frames that are received periodically, at random time intervals, or at each time a specific event occurs. For example, when a scan operation is resumed after a pause, the data processing apparatus 120 may identify the type of an object using a predetermined number of frames among images received after the scan operation is resumed.

In an embodiment, the data processing apparatus 120 may identify an object to be filtered based on the scan filtering mode.

In an embodiment, the data processing apparatus 120 may identify an object to be filtered using the neural network model 322.

According to an embodiment, in a state in which the scan filtering mode is activated, the neural network model 322 may identify a class to be filtered in a selected activation mode among pixels included in frames received from the 3D scanner 110. In an embodiment, the data processing apparatus 120 may obtain filtered 3D scan data on an object by removing pixels identified by the neural network model 322.

For example, when the scan filtering mode is the first activation mode, the neural network model 322 may identify the type of an object based on the positional relationship or characteristics of objects included in the input image 410 and may determine regions other the teeth, gingivae, and palate as objects to be filtered. The data processing apparatus 120 may filter regions other than teeth, gingivae, and palate that are identified by the neural network model 322.

For example, when the scan filtering mode is the second activation mode, the neural network model 322 may identify, as objects to be filtered, regions other than the teeth and gingivae among objects included in the input image 410. The data processing apparatus 120 may filter all tissues other than the teeth and gingivae identified by the neural network model 322.

For example, when the scan filtering mode is the third activation mode, the neural network model 322 may identify, as objects to be filtered, regions other than the teeth among objects included in the input image 410. The data processing apparatus 120 may filter all regions other than the teeth identified by the neural network model 322.

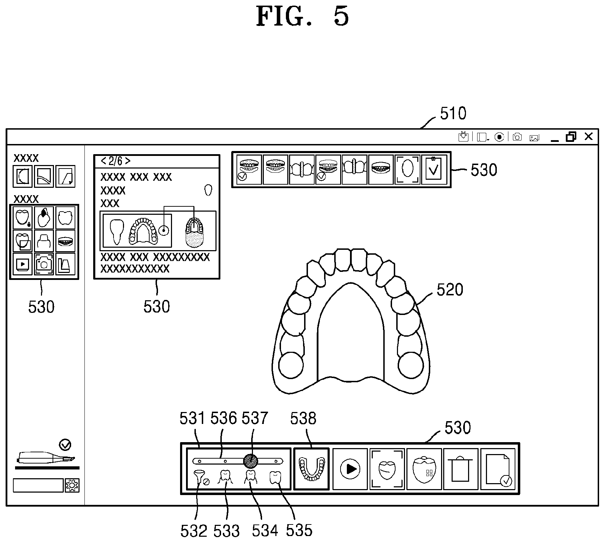

FIG. 5 is a diagram illustrating a screen 510 output from the data processing apparatus 120 according to an embodiment.

In FIG. 5, the screen 510 is output from the data processing apparatus 120 when the data processing apparatus 120 executes dedicated scan software connected to the 3D scanner 110. The data processing apparatus 120 may obtain 3D scan data 520 from an image received from the 3D scanner 110 and may output the obtain 3D scan data 520 on the screen 510.

In an embodiment, the data processing apparatus 120 may output a menu bar 530 including various menus on the screen 510. The menu bar 530 may include information on shortcut keys or data-manipulation keys that the user may input through the user input unit 125 such as a keyboard or a mouse provided in the data processing apparatus 120.

The menu bar 530 may include commands or methods for operating the data processing apparatus 120. The menu bar 530 may be implemented in various forms such as icons, text, or images. The menu bar 530 may include various toolbars for editing or changing scan data using the data processing apparatus 120. For example, the menu bar 530 may include menus such as enlarging or reducing scan data, viewing in a full screen, viewing previous images, or changing angles or positions. Alternatively, the menu bar 530 may display whether the scan mode is a maxilla scan mode, a mandible scan mode, or an occlusion scan mode, or may include a menu for selecting a scan mode. Alternatively, the menu bar 530 may include a menu for completing the scan mode.

In an embodiment, the menu bar 530 output by the data processing apparatus 120 may include a scan filtering mode menu 531. In an embodiment, the scan filtering mode menu 531 may be a menu for allowing a user to select a scan filtering mode. Alternatively, the scan filtering mode menu 531 may inform a user of the currently-set scan filtering mode.

In an embodiment, the scan filtering mode menu 531 may include a menu for selecting whether to activate the scan filtering mode.

In an embodiment, the scan filtering mode may include a plurality of activation modes depending on targets or regions to be filtered. In an embodiment, the scan filtering mode menu 531 may include a menu for selecting one of the activation modes of the scan filtering mode for performing a scan filtering operation.

In an embodiment, the scan filtering mode may be divided into three modes depending on regions to be filtered. A first activation mode may refer to a mode for filtering out soft tissue other than only the teeth, gingiva, and palate. In an embodiment, a second activation mode may refer to a mode for filtering out tissue other than the teeth and gingiva. In an embodiment, a third activation mode may refer to a mode for leaving only the teeth.

In embodiments, one of the scan filtering modes may be selected in various manners. For example, when the data processing apparatus 120 is connected to the 3D scanner 110, deactivating the scan filtering mode may be selected as a default, or one of the activation modes may be selected as a default.

Alternatively, in an embodiment, the data processing apparatus 120 may automatically retrieve an activation mode used in the previous scan operation and select the activation mode as the current activation mode. For example, when the scan filtering mode is deactivated in the previous scan operation, the data processing apparatus 120 may automatically select deactivation of scan filtering mode deactivation in the current scan operation. Alternatively, when one of the activation modes of the scan filtering mode, for example, the second activation mode, is used in the previous scan operation, the data processing apparatus 120 may automatically select the second activation mode of the scan filtering mode in the current scan operation.

Alternatively, in an embodiment, a user may select deactivation of the scan filtering mode or directly select one of the activation modes of the scan filtering mode. When a user desires an activation mode other than a default activation mode or an activation mode used in the immediately previous scan operation, the user may directly select the desired activation mode for operation in the desired activation mode.

Referring to FIG. 5, the scan filtering mode menu 531 may include a menu 532 for deactivating the scan filtering mode and menus (menus 533 to 535) for activating the scan filtering mode. The menus for activating the scan filtering mode may include a menu 533 for selecting the first activation mode, a menu 534 for selecting the second activation mode, and a menu 535 for selecting the third activation mode. Each of the menus may be expressed with an icon, text, or an image.

In an embodiment, the scan filtering mode menu 531 may further include an identifier 537 that indicates a mode selection or a selected mode. The identifier 537 may be output on a bar 536 at a position corresponding to a selected mode menu to show a user the currently selected scan filtering mode.

In an embodiment, a user may manipulate the identifier 537 included in the scan filtering mode menu 531 using the user input unit 125 such as a keypad, a keyboard, a mouse, or a touch pad to select a desired scan filtering mode. For example, a user may select a desired scan filtering mode by selecting the identifier 537 using the user input unit 125 and move the identifier 537 along the bar 536 to a position corresponding to a desired mode menu. Alternatively, when a user selects a desired mode menu or selects a position of on the bar 536 corresponding to the desired mode menu, the identifier 537 may move to the position of the bar 536 corresponding to the mode menu selected by the user, and the mode menu may be selected.

In an embodiment, the menu bar 530 may include an impression scan menu 538. A user may select the impression scan menu 538 to scan an impression.

In an embodiment, the data processing apparatus 120 may scan an object, invert scan data after the object is completely scanned, and output results of the inversion on the screen 510.

In an embodiment, when a user selects the impression scan menu 538, the data processing apparatus 120 may identify an object as an impression. Thus, when the impression scan menu 538 is selected, the data processing apparatus 120 may generate 3D scan data on the object by deactivating the scan filtering mode.

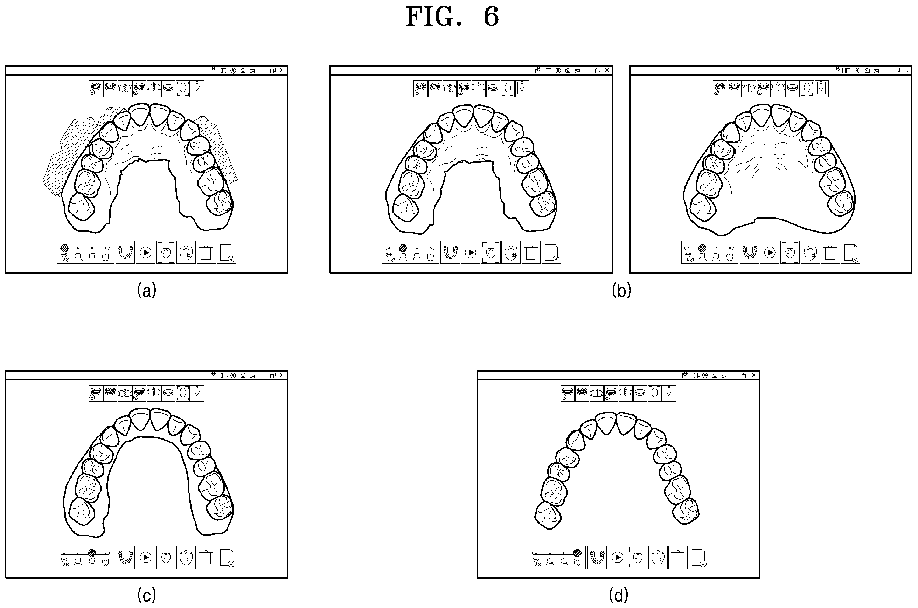

FIGS. 6A to 6D are diagrams illustrating 3D scan data acquired by the data processing apparatus 120 based on various scan filtering modes according to embodiments.

FIG. 6A shows 3D scan data acquired by the data processing apparatus 120 when a scan filtering mode is deactivated.

In some cases, a user may want to acquire 3D scan data without filtering. For example, when an object is an edentulous jaw, the object often does not contain teeth, and only soft tissue may remain on the object. It may be difficult for the data processing apparatus 120 to distinguish between the gingiva and the mucous membrane in an image containing only soft tissue, the object may be misidentified as another tissue. In other words, there is a possibility that the data processing apparatus 120 mistakenly determines necessary tissue data included in an image of the object as unnecessary tissue data and removes the necessary tissue data. Therefore, in this case, a user may attempt to obtain 3D scan data on the edentulous jaw without filtering.

In an embodiment, when a user selects deactivation of the scan filtering mode, the data processing apparatus 120 may generate scan data from images of an object without filtering, and thus, the scan data may not be filtered.

In an embodiment, when the data processing apparatus 120 identifies the type of an object as a model, the data processing apparatus 120 may automatically deactivate a scan filtering operation and generate scan data. When the type of an object is a model, the object may include at least one of a plaster model, an impression, and a dentiform.

Unlike the actual oral cavity, a model does not contain unnecessary tissue that may affect generation of scan data, and 3D scan data on an object may be more accurately obtained as the number of images of the object increases. Thus, in an embodiment, when the type of an object is a model, the data processing apparatus 120 may deactivate a scan filtering operation and obtain 3D scan data on the object.

FIGS. 6B to 6D each show 3D scan data acquired by the data processing apparatus 120 when the scan filtering mode is activated.

FIG. 6B shows 3D scan data acquired by the data processing apparatus 120 when the scan filtering mode is a first activation mode. In an embodiment, the first activation mode may refer to a mode for filtering out soft tissue other than the teeth, gingivae, and palate. The first activation mode may be a common activation mode in most cases. In an embodiment, when the first activation mode is selected, the data processing apparatus 120 may filter out tissue other than the teeth, gingivae, and palate.

FIG. 6B shows two screens. Both the screens in FIG. 6B include regions of the teeth, gingivae, and palate. The left and right screens in FIG. 6B show that a user has scanned the palate to different degrees with the 3D scanner 110. The left screen in FIG. 6B shows 3D scan data obtained by the data processing apparatus 120 in the first activation mode when an object scanned by a user with the 3D scanner 110 does not include much of the palate. The right screen in FIG. 6B shows 3D scan data obtained by the data processing apparatus 120 in the first activation mode when an object scanned by a user with the 3D scanner 110 includes the entire palate.

FIG. 6C shows 3D scan data acquired by the data processing apparatus 120 when the scan filtering mode is a second activation mode.

In an embodiment, unlike the first activation mode, the second activation mode may refer to a mode for filtering out regions other than only the teeth and gingivae. The 3D scan data generated in the second activation mode includes only the teeth and gingivae, and thus, a user does not need to trim unnecessary soft tissue data using the data processing apparatus 120.

FIG. 6D shows 3D scan data acquired by the data processing apparatus 120 when the scan filtering mode is a third activation mode. In an embodiment, the third activation mode may refer to a mode for filtering out all soft tissue around the teeth and leaving only the hard tissue, that is, the teeth. In the third activation mode, the data processing apparatus 120 acquires 3D scan data from which all regions other than the teeth are removed. Although 3D scan data on an object is acquired in the first or second activation mode, the 3D scan data includes an empty region of the teeth, a user may additionally scan the empty region in the third activation mode. 3D scan data generated in the third activation mode may be aligned and merged with the 3D scan data generated in the first or second activation mode.

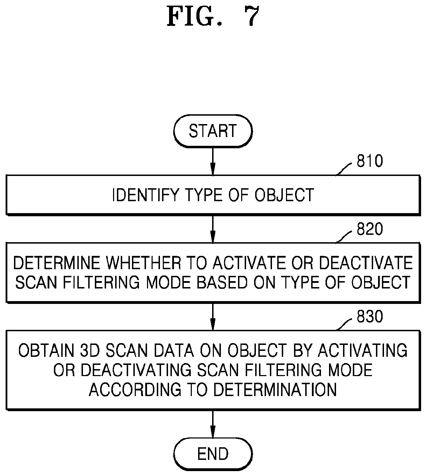

FIG. 7 is a diagram illustrating a user interface screen 730 output by the data processing apparatus 120 for allowing changing of the current activated or deactivated state of a scan filtering mode according to an embodiment.

Referring to FIG. 7, the data processing apparatus 120 may output a scan filtering mode menu 710. The scan filtering mode menu 710 may include a menu 711 for deactivating the scan filtering mode and a menu 712 for selecting a first activation mode of the scan filtering mode. Additionally, the scan filtering mode menu 531 may include an identifier 714 for selecting a mode and/or indicating a selected mode and a bar 713 along which the identifier 714 may be moved.

In an embodiment, the data processing apparatus 120 may identify the current activated or deactivated state of the scan filtering mode.

Referring to FIG. 7, the identifier 714 is currently located on the bar 713 at a position corresponding to the first activation mode selection menu 712, indicating that the scan filtering mode is currently set to the first activation mode. The data processing apparatus 120 may identify that the scan filtering mode is currently active.

In an embodiment, the data processing apparatus 120 may identify the type of an object in an image acquired in real time from the 3D scanner 110. In an embodiment, the data processing apparatus 120 may determine to deactivate the scan filtering mode when the type of the object is detected as a plaster model or an impression model.

In an embodiment, when the current activated or deactivated state of the scan filtering mode does not correspond to the type of the object, the data processing apparatus 120 may automatically change the current activated or deactivated state of the scan filtering mode.

Alternatively, in an embodiment, when the current activated or deactivated state of the scan filtering mode does not correspond to the type of the object, the data processing apparatus 120 may not automatically change the current activated or deactivated state of the scan filtering mode but may output an interface screen informing a user of the situation to allow the user to change the mode.

FIG. 7 illustrates an embodiment in which when the current activated or deactivated state of the scan filtering mode does not correspond to the type of an object, the data processing apparatus 120 outputs the user interface screen 730 for changing the current activated or deactivated state of the scan filtering mode.

In an embodiment, the data processing apparatus 120 may display the user interface screen 730 in the form of a text window in a region of a screen. The size, output location, transparency, and/or shape of the user interface screen 730 may be modified in various manners.

In an embodiment, the user interface screen 730 may include at least one of an identified type of an object, a message for a user to change the activated or deactivated state of the scan filtering mode according to the identified type of the object, and a message for a user to change a scan operation. However, the user interface screen 730 is not limited thereto and may display variously modified messages.

When the user interface screen 730 is output, a user may input a signal for changing the activated or deactivated state of the scan filtering mode according to information provided by the user interface screen 730. For example, a user may select the menu 711 of the scan filtering mode menu 710 to deactivate the scan filtering mode. The data processing apparatus 120 may receive the signal that is input in response to the output of the user interface screen 730 for changing the activated or deactivated state of the scan filtering mode and may change the current activated or deactivated state of the scan filtering mode based on the signal.

Alternatively, when an object is an impression, a user may select an impression scan menu 720. In an embodiment, the data processing apparatus 120 may identify the object as an impression model when the impression scan menu 720 is selected by a user. In an embodiment, the data processing apparatus 120 may automatically deactivate the scan filtering mode in response to the selection of the impression scan menu 720 and may perform an impression scan operation on the object. In an embodiment, when the object is completely scanned, the data processing apparatus 120 may perform a relief-intaglio inversion on scan data and may output results of the relief-intaglio inversion.

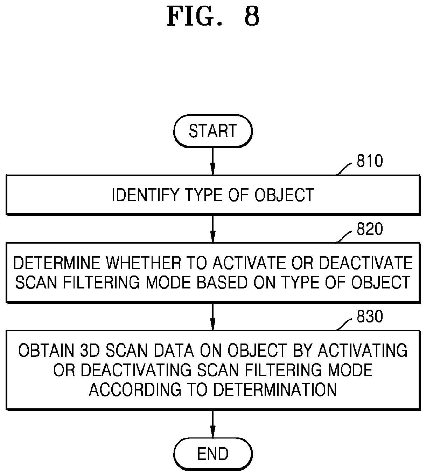

FIG. 8 is a flowchart illustrating a data processing method according to an embodiment.

Referring to FIG. 8, the data processing apparatus 120 may identify the type of an object (operation 810). For example, the data processing apparatus 120 may determine whether the type of the object is a model.

In an embodiment, the data processing apparatus 120 may determine whether to activate or deactivate a scan filtering mode based on the type of the object (operation 820). For example, the data processing apparatus 120 may determine that the scan filtering mode is to be deactivated when the type of the object is a model. For example, the data processing apparatus 120 may determine that the scan filtering mode is to be activated when the type of the object is the oral cavity rather than a model.

In an embodiment, the data processing apparatus 120 may obtain 3D scan data on the object by activating or deactivating the scan filtering mode according to the determination (operation 830).

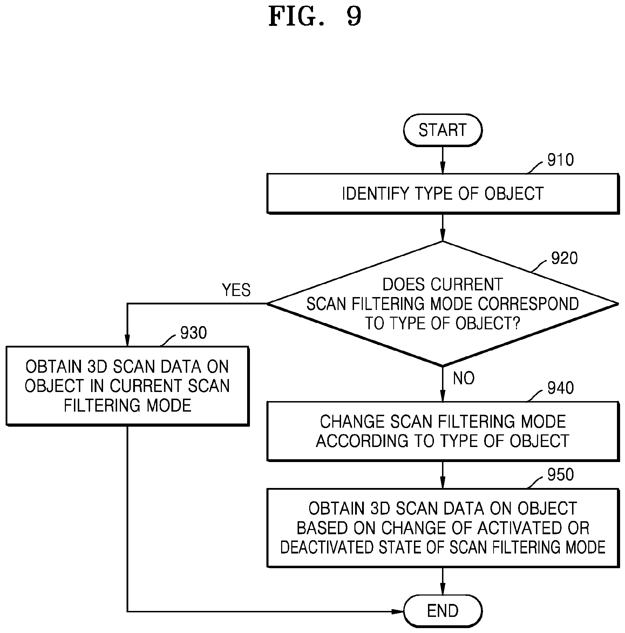

FIG. 9 is a flowchart illustrating a data processing method according to an embodiment.

Referring to FIG. 9, the data processing apparatus 120 may identify the type of an object (operation 910).

In an embodiment, the data processing apparatus 120 may determine whether the current scan filtering mode corresponds to the type of the object (operation 920).

For example, when the data processing apparatus 120 determines that the type of the object is a model such as a plaster model, an impression model, or a dentiform, the data processing apparatus 120 may determine that the type of the object corresponds to deactivation of the scan filtering mode. For example, when the type of the object is not a model, the data processing apparatus 120 may determine that the type of the object corresponds to activation of the scan filtering mode.

In an embodiment, when the data processing apparatus 120 determines that the current scan filtering mode corresponds to the type of the object, 3D scan data on the object may be obtained in the current scan filtering mode (operation 930).

In an embodiment, when the data processing apparatus 120 determines that the current scan filtering mode does not correspond to the type of the object, the data processing apparatus 120 may change the scan filtering mode according to the type of the object (operation 940).

For example, when the data processing apparatus 120 determines that the current scan filtering mode does not correspond to the type of the object, the data processing apparatus 120 may automatically change the scan filtering mode according to the type of the object.

Alternatively, when the data processing apparatus 120 determines that the current scan filtering mode does not correspond to the type of object, the data processing apparatus 120 may output a user interface screen indicating the determination and may change the scan filtering mode according to the type of the object based on a signal that is input by a user in response to the user interface screen for changing the activated or deactivated state of the scan filtering mode.

In an embodiment, the data processing apparatus 120 may obtain 3D scan data on the object based on the change of the activated or deactivated state of the scan filtering mode (operation 950).

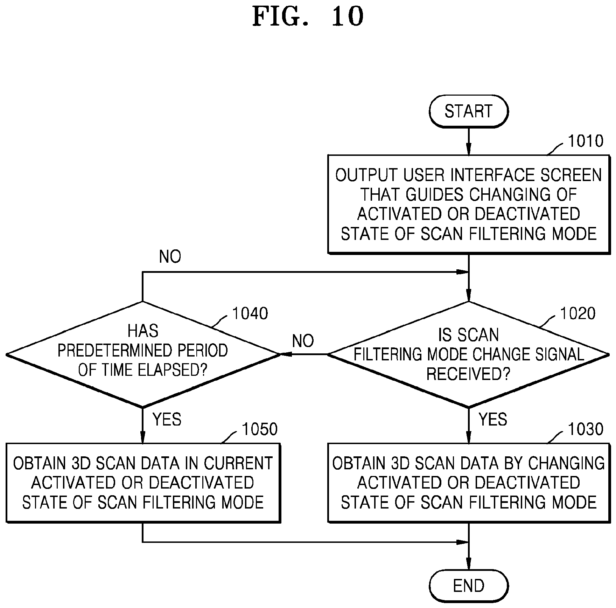

FIG. 10 is a flowchart showing a data processing method according to an embodiment.

Referring to FIG. 10, the data processing apparatus 120 may determine whether the current scan filtering mode corresponds to the type of an object.

For example, when the data processing apparatus 120 determines that an identified type of the object is a plaster model and the current scan filtering mode is active, the data processing apparatus 120 may determine that the current scan filtering mode does not correspond to the type of the object.

When the data processing apparatus 120 determines that the current scan filtering mode does not correspond to the type of object, the data processing apparatus 120 may output a user interface screen to guide changing of the activated or deactivated state of the scan filtering mode (operation 1010).

A user may be informed, through the user interface screen output by the data processing apparatus 120, that the scan filtering mode is not correctly activated or deactivated for the object to be scanned right now, and thus, the user may change the activated or deactivated state of the scan filtering mode. For example, as in the example described above, when the object to be currently scanned is a plaster model, the user may input a mode change signal requesting deactivation of the scan filtering mode.

In an embodiment, the data processing apparatus 120 may determine whether a mode change signal is received from a user in response to the output of the user interface screen (operation 1020).

In an embodiment, the data processing apparatus 120 may obtain 3D scan data by changing the activated or deactivated state of the scan filtering mode based on the mode change signal that requests changing of the activated or deactivated state of the scan filtering mode (operation 1030). For example, in the example described above, the data processing apparatus 120 may change the scan filtering mode from an activated state to a deactivated state and may obtain 3D scan data on the plaster model.

In an embodiment, when the data processing apparatus 120 does not receive a mode change signal that requests changing of the activated or deactivated state of the scan filtering mode, the data processing apparatus 120 may determine whether a predetermined period of time has elapsed (operation 1040).

In an embodiment, when the data processing apparatus 120 does not receive a mode change signal requesting changing of the activated or deactivated state of the scan filtering mode after a predetermined period of time from the output of the user interface screen, the data processing apparatus 120 may obtain 3D scan data according to the current activated or deactivated state of the scan filtering mode (operation 1050). For example, the data processing apparatus 120 may obtain 3D scan data on a plaster model in a state in which the scan filtering mode is activated.

In an embodiment, the data processing method may be implemented in the form of program instructions executable by various computers and may be recorded on a computer-readable recording medium. Also, an embodiment may provide a computer-readable recording medium on which at least one program including at least one instruction for executing the data processing method is recorded.

In addition, according to the embodiments described above, the data processing method may include: identifying a type of an object; determining, based on the type of the object, whether to activate or deactivate a scan filtering mode; and acquiring 3D scan data on the object by activating or deactivating the scan filtering mode according to the determining, and the data processing method may be implemented as a computer program product including a computer-readable recording medium on which a program for implementing the data processing method is recorded.

The computer-readable recording medium may include program instructions, data files, data structures or the like alone or in combination. Examples of the computer-readable recording medium may include: magnetic media such as hard disks, floppy disks, and magnetic tapes; optical recording media such as CD-ROMs and DVDs; magnetooptical media such as floptical disks; and hardware such as ROMs, RAMs, and flash memories specifically configured to store program instructions and execute the program instructions.

Here, the computer-readable recording medium may be provided in the form of non-transitory recording media. Here, the "non-transitory recording media" may refer to tangible recording media. Also, the "non-transitory recording media" may include buffers in which data is temporarily stored.

In some embodiments, the data processing method according to the various embodiments described above may be included in a computer program product. The computer program product may be distributed in the form of a machine-readable recording medium (for example, compact disc read only memory (CD-ROM)). In addition, the computer program product may be distributed online (for example, downloaded or uploaded) through application stores (for example, Play Store, etc.) or directly between two user devices (for example, smartphones). According to embodiments, for example, the computer program product may include a recording medium on which a program including at least one instruction for executing the data processing method is recorded.

While embodiments have been described in detail, the scope of the present disclosure is not limited to the embodiments, and various modifications and improvements may be made by those of ordinary skill in the art using the basic concept of the present disclosure as defined in the following claims.

## Claims

1. A data processing method performed by a data processing apparatus, the data processing method comprising:
identifying a type of an object;
determining, based on the type of the object, whether to activate or deactivate a scan filtering mode; and
obtaining 3D scan data on the object by activating or deactivating the scan filtering mode according to the determining.

2. The data processing method of claim 1, wherein the determining of, based on the type of the object, whether to activate or deactivate the scan filtering mode comprises:
based on the type of the object being a model, determining to deactivate the scan filtering mode; and
based on the type of the object not being a model, determining to activate the scan filtering mode.

3. The data processing method of claim 1, wherein the obtaining of the 3D scan data on the object comprises:
changing a current activated or deactivated state of the scan filtering mode based on the current activated or deactivated state of the scan filtering mode not corresponding to the determining; and
obtaining 3D scan data on the object based on the changed activated or deactivated state of the scan filtering mode.

4. The data processing method of claim 3, further comprising outputting a user interface screen for changing the current activated or deactivated state of the scan filtering mode based on the current activated or deactivated state of the scan filtering mode not corresponding to the determining,
wherein the user interface screen comprises at least one of the type of the object, a message guiding changing of the activated or deactivated state of the scan filtering mode based on the type of the object, and a message guiding changing of a scan operation.

5. The data processing method of claim 4, wherein the changing of the current activated or deactivated state of the scan filtering mode comprises:
receiving a signal for changing the current activated or deactivated state of the scan filtering mode, the signal being input in response to the outputting of the user interface screen; and
changing the current activated or deactivated state of the scan filtering mode based on the signal.

6. The data processing method of claim 1, wherein the identifying of the type of the object comprises identifying the object as a specific class when a percentage of pixels identified as the specific class among all pixels included in a frame received from a scanner is greater than or equal to a reference value.

7. The data processing method of claim 6, wherein the identifying of the type of the object further comprises
identifying the object as the specific class when a percentage of frames identified as the specific class among a reference number of frames obtained after a scan operation starts is greater than or equal to a reference value.

8. The data processing method of claim 1, wherein the scan filtering mode comprises a plurality of activation modes depending on objects to be filtered,
the obtaining of the 3D data on the object by activating the scan filtering mode comprises obtaining filtered 3D scan data on the object in one activation mode among the plurality of activation modes, and
the one activation mode among the plurality of activation modes comprises at least one of an activation mode selected by a user, an activation mode selected by default, and an activation mode used in a previous scan operation.

9. The data processing method of claim 8, wherein the obtaining of the filtered 3D scan data on the object in the one activation mode of the plurality of activation modes comprises:
identifying pixels of a class to be filtered in the one activation mode among pixels included in a frame received from a scanner; and
obtaining filtered 3D scan data on the object by removing pixels of the identified class.

10. A computer-readable recording medium having recorded thereon a program for executing a data processing method, the data processing method comprising:
identifying a type of an object;
determining, based on the type of the object, whether to activate or deactivate a scan filtering mode; and
obtaining 3D scan data on the object by activating or deactivating the scan filtering mode according to the determining.

11. A data processing apparatus comprising at least one processor configured to execute at least one instruction,
wherein the at least one processor is further configured to execute the at least one instruction to:
identify a type of an object;
determine, based on the type of the object, whether to activate or deactivate a scan filtering mode; and
obtain 3D scan data on the object by activating or deactivating the scan filtering mode according to the determining.

12. The data processing apparatus of claim 11, wherein the at least one processor is further configured to execute the at least one instruction to determine, based on the type of the object being a model, to deactivate the scan filtering mode.

13. The data processing apparatus of claim 11, wherein the at least one processor is further configured to execute the at least one instruction and to determine, based on the type of object being not a model, to activate the scan filtering mode.

14. The data processing apparatus of claim 11, wherein the at least one processor is further configured to execute the at least one instruction to:
change a current activated or deactivated state of the scan filtering mode based on the current activated or deactivated state of the scan filtering mode not corresponding to the determining; and
obtain 3D scan data on the object based on the changed activated or deactivated state of the scan filtering mode.

15. The data processing apparatus of claim 14, further comprising a display, wherein the at least one processor is further configured to execute the at least one instruction to output a user interface screen for changing the current activated or deactivated state of the scan filtering mode based on the current activated or deactivated state of the scan filtering mode not corresponding to the determining,
wherein the user interface screen comprises at least one of the type of the object, a message guiding changing of the activated or deactivated state of the scan filtering mode based on the type of the object, and a message guiding changing of a scan operation.

16. The data processing apparatus of claim 15, further comprising a user input unit,
wherein the at least one processor is further configured to execute the at least one instruction to:
receive a signal for changing the activated or deactivated state of the scan filtering mode, the signal being input through the user interface screen in response to the outputting of the user interface screen; and
change the current activated or deactivated state of the scan filtering mode based on the signal.

17. The data processing apparatus of claim 11, wherein the at least one processor is further configured to execute the at least one instruction to identify the object as a specific class when a percentage of pixels identified as the specific class among all pixels included in a frame received from a scanner is greater than or equal to a reference value.

18. The data processing apparatus of claim 17, wherein the at least one processor is further configured to execute the at least one instruction to identify the object as the specific class when a percentage of frames identified as the specific class among a reference number of frames obtained after a scan operation starts is greater than or equal to a reference value.

19. The data processing method of claim 11, wherein the scan filtering mode comprises a plurality of activation modes depending on objects to be filtered,
the at least one processor is further configured to execute the at least one instruction to obtain filtered 3D scan data on the object in one activation mode among the plurality of activation modes, and
the one activation mode among the plurality of activation modes comprises at least one of an activation mode selected by a user, an activation mode selected by default, and an activation mode used in a previous scan operation.

20. The data processing apparatus of claim 19, wherein the at least one processor is further configured to execute the at least one instruction to:
identify pixels of a class to be filtered in the one activation mode among pixels included in a frame received from a scanner; and
obtain filtered 3D scan data on the object by removing pixels of the identified class.
